# EUROPEAN PATENT SPECIFICATION

(11) **EP 2 326 341 B1**
(45) Date of publication and mention of the grant of the patent: **04.07.2012**
(21) Application number: 09788312.8
(22) Date of filing: 09.09.2009
(51) Int. Cl.: A61K 38/11

(54) **Oxytocin formulations and uses thereof**
Oxytocin-Formulierungen und ihre Verwendungen
Formulations d'oxytocine et leur utilisations

(30) Priority: 09.09.2008 EP 08163932
(43) Date of publication of application: 01.06.2011
(73) Proprietor: Rijksuniversiteit Groningen, 9712 CP Groningen (NL)
(72) Inventor: AMORIJ, Jean-Pierre, NL-1544 PA Zaandijk (NL); AVANTI, Christina, NL-9725 AN Groningen (NL); FRIJLINK, Henderik, Willem, NL-9761 KM Eelde (NL); HINRICHS, Wouter, Leonardus, Joseph, NL-9718 EG Groningen (NL)
(74) Representative: Jansen, Cornelis Marinus
(86) International application number: PCT/NL2009/050542
(87) International publication number: WO 2010/030180

(56) References cited:
- MX-A- 9 707 899
- KALIS ET AL: LATVIJAS PSR ZINATNU AKADEMIJAS VESTIS, KIMIJAS SERIJA, vol. 29, no. 1, 1970, pages 29-32, XP008101303 cited in the application
- TRISSEL ET AL: INTERNATIONAL JOURNAL OF PHARMACEUTICAL COMPOUNDING, vol. 10, 2006, pages 156-158, XP002513262 cited in the application
- BOOTHBY ET AL: "Extended stability of oxytocin in Ringer's lactate solution at 4° and 25°C" HOSPITAL PHARMACY, vol. 41, 2006, pages 437-441, XP002513263
- DE GROOT ET AL: "Oxytocin and desamino-oxytocin tablets are not stable under simulated tropical conditions" JOURNAL OF CLINICAL PHARMACY AND THERAPEUTICS, vol. 20, 1995, pages 115-119, XP002513264 cited in the application
- WYTTENBACH ET AL: "Interactions of the hormone oxytocin with divalent metal ions" JOURNAL OF THE AMERICAN CHEMICAL SOCIETY, vol. 130, May 2008 (2008-05), pages 5993-6000, XP002513366
- BAL ET AL: "Potentiometric and spectroscopic studies of the Cu(II) complexes of Ala-Arg(8)-vasopressin and oxytocin: Two vasopressin-like peptides" JOURNAL OF INORGANIC BIOCHEMISTRY, vol. 45, 1992, pages 193-202, XP002513265
- ANANTHANARAYANAN ET AL: "Interaction of oxytocin with Ca(2+): I. CD and fluorescence spectral characterization and comparison with vasopressin" BIOPOLYMERS, vol. 40, 1996, pages 433-443, XP002513367
- SLANINOVÁ ET AL: "Oxytocin, divalent cations and mechanism of action" JOURNAL OF PEPTIDE SCIENCE, vol. 12, no. S1, 2006, page 404, XP002590740
- LIU ET AL: "Oxytocin-receptor binding: Why divalent metals are essential" JOURNAL OF THE AMERICAN CHEMICAL SOCIETY, vol. 127, 2005, pages 2024-2025, XP002590741
- BRASUN ET AL: "Histidine analogues of oxytocin and vasopressin as efficient ligands for Zn2+ ions - potentiometric and NMR studies" JOURNAL OF INORGANIC BIOCHEMISTRY, vol. 103, 10 May 2009 (2009-05-10), pages 1033-1038, XP026218206

## Description

The present invention relates to the field of preventive and therapeutic medicine. In particular, it relates to therapeutic formulations comprising disulfide bridge-containing neurohypophysial nonapeptides, such oxytocin, vasopressin or analogs thereof. Provided are heat-stable oxytocin formulations and uses thereof in indications such as induction of labor, augmentation of labor, post-partum haemorrhage or uterine atony. Also provides are heat-stable vasopressin formulations and uses thereof in indications such as diabetes insipidus and vasodilatory shock.

Oxytocin (Cys-Tyr-Ile-Gln-Asn-Cys-Pro-Leu-Gly-NH₂) and vasopressin (Cys-Tyr-Phe-Gln-Asn-Cys-Pro-Arg-Gly-NH₂) are peptide hormones that are produced in the brain of most mammals, including humans, and that share a high degree of homology. Both nonapeptides originate from the same evolutionary progenitor, vasotocin (Cys-Tyr-Ile-Gln-Asn-Cys-Pro-Arg-Gly-NH₂). Their respective amino acid sequences differ only at position 3 (Ile versus Phe) and position 8 (Leu versus Arg). Both peptides contain a sulfur bridge that is formed by the cysteine residues at positions 1 and 6.

Next to their structural resemblance, there is also an anatomical relationship between oxytocin and vasopressin (also known as arginine vasopressine (AVP), argipressin and anti-diuretic hormone (ADH)). For example, in most species the genes for these peptides are located on the same chromosome and are separated by a relatively small distance of less than 15.000 bases. Also, the hypothalamic magnocellular neurons that synthesize oxytocin are located adjacent to those that synthesize vasopressin, and are similar in many respects.

Furthermore, it is known that the structural similarity of these hormones causes some functional cross-reactions in the body: whereas oxytocin has a slight antidiuretic function, high levels of vasopressin may cause uterine contractions.

Oxytocin, vasopressin and analogues thereof are widely used in human and veterinarian medicine. Vasopressin is mainly applied for treatment of diabetes insipidus and vasodilatory shock, the latter occurring for example during sepsis, organ transplantation or after implantation of a cardiopulmonary bypass. It is commercially available as either generic vasopressin or synthetic analogues, such as desmopressin (1-desamino-8-D-arginine vasopressin or DDAVP; trade names Stimate®, Minirin® and Octostim®) and terlipressin (trade name Glypressin®). Oxytocin is often used to induce labor and support labor in case of non-progression of parturition and to treat (post-partum) haemorrhage. Currently, oxytocin is considered to be the principal agent to treat post-partum haemorrhage. It is degraded in the gastrointestinal tract, therefore it is administered as aqueous formulation by injection or as nasal spray. Its half-life in the blood is typically about three minutes. Synthetic oxytocin is commercially available as ready-to-use aqueous formulations under the trade names Pitocin® and Syntocinon® or as generic oxytocin. The oxytocin analogues desamino-oxytocin and carbetocin (trade name Duratocin®) are also commercially available.

According to the World Health Report 2005 as issued by the World Health Organization, in Africa, Asia and Latin America each year half a million women die as a result of problems during pregnancy and childbirth. In Africa and Asia, at least 25% of those deaths can be attributed to haemorrhage, most commonly caused by failure of the uterus to contract adequately after childbirth (atonicity). This makes haemorrhage the leading cause of maternal deaths in these continents (Khan et al., Lancet 367 (9516): 1066-1074, 2006). In third world countries, it is often practically and/or economically impossible to protect pharmaceutical preparations from the harmful effects of high temperatures during transportation, storage and use. Reports and stability studies of injectable oxytocins have shown serious instability on exposure to increased temperatures and exposure to light (see de Groot et al., World Health Organization, WHO/DAP/94.13, 1994 and de Groot et al., J Clin Pharm Ther 20 (2): 115-119, 2008, and references cited therein). The 1994 publication by de Groot *et al*. discloses that tablets of oral oxytoxin or analogs thereof (also referred to as oxytocics) such as ergometrine, methylergometrine, oxytocin and desamino-oxytocin, are not stable under simulated tropical conditions, and concludes that oral oxytocins are not suitable for use in the prevention of postpartum haemorrhage. Thus, despite the availability of various formulations of oxytocin and desamino-oxytocin for treatment of haemorrhage, the (sub)tropical ambient temperatures and the absence of a so-called cold-chain in these parts of the world severely affect their stability and functionality. Proper prophylaxis and / or treatment of haemorrhage is therefore nearly impossible, resulting in this astonishingly high number of casualties each year. Vasopressin formulations known to date are similarly affected by exposure to high ambient temperature or light.

Given the reduced stability of formulations comprising bioactive or therapeutic peptides such as oxytocin or vasopressin at increased temperatures, their use in treatment methods in (sub)tropical countries, including many third world countries, is limited. Thus, there is a clear need for peptide formulations, preferably as a ready-to-use injectable aqueous solution, that have an improved thermal stability.

One object of the invention to provide means and methods to increase the thermal stability of aqueous formulations comprising oxytocin, vasopressin or an analogue thereof. A further object is the provision of a therapeutic or prophylactic oxytocin- or vasopressin- formulation, e.g. a ready-to-use medication that can be administered to a subject in need thereof, that has a stability that can withstand (sub)tropical ambient temperatures, is cheap to produce and / or is free of side effects. In a specific aspect, the invention aims to provide an injectable formulation comprising oxytocin or vasopressin displaying improved thermal stability as compared to existing formulations.

The present inventors surprisingly discovered that the stability of aqueous peptide formulations is greatly enhanced by the presence of a buffer and at least one non-toxic source of divalent metal ions in a concentration of at least 2 mM. For example, aqueous oxytocin formulations show much less degradation when kept at 66 °C for at least 4 weeks when formulated with divalent metal ions in a concentration of at least 2 mM in the presence of a buffer. Preferably, the source of divalent metal ions is a metal salt.

This is an important finding since until now increased stability of small therapeutic Cys-containing peptides such as oxytocin could only be obtained at non-tropical conditions. For example, when dissolving oxytocin in the common NaCl infusion solution, oxytocin stability was only observed at temperatures of either near 23 °C (Trissel et al., Int J Pharm Comp 2006 10(2): 156-188) or 36 °C (Kalis et al., Latvijas Psr Zinatnu Akademijas Vestis, Kimijas Serija 1970 29(1): 29-32).

Disclosed is a pH-buffered aqueous formulation comprising a therapeutically effective amount of oxytocin, vasopressin or an analogue thereof and at least one non-toxic (i.e. biocompatible) source of divalent metal ions in a concentration of at least 2 mM. Provided is an aqueous formulation according to claim 1. Such formulations are not known in the art. Trissel *et al*. demonstrate that dissolving oxytocin) in another common infusion solution, i.e. commercially obtainable lactated Ringer's solution, had no effect on oxytocin stability at all. Ringer's lactate solution does not contain at least 2 mM divalent metal ions, When kept at room temperature the stability of oxytocin in Ringer's was already compromised after 30 days, even at an oxytocin concentration as low as 0.08 IU/ml.

MX 9 707 899 discloses the use of sodium and/or potassium ions in combination with one or more anions to stabilize formulations comprising synthetic vasopressin or oxytocin analogues. It teaches a strong preference for a mixture or citric acid, phosphate and a sodium ions. Buffered formulations comprising at least 2 mM divalent metal ions are not disclosed.

As mentioned above, the presence of at least 2 mM divalent metal ions and a buffer greatly enhances the stability of injectable aqueous oxytocin solutions. The invention accordingly relates to the use of a combination of a buffer and at least one non-toxic source of divalent metal ions in a concentrations of at least 2 mM for stabilizing aqueous formulations comprising oxytocin or vasopressin or an analogue thereof.

Furthermore, it relates to stabilized oxytocin formulations and use thereof in methods for the prophylaxis or treatment or haemorrhage in a subject in need thereof. It also relates to stabilized vasopressin formulations and use thereof in methods for the prophylaxis or treatment of diabetes insipidus or vasodilatory shock in a subject in need thereof.

Also provided is the use of a stabilized oxytocin formulation for the manufacture of a medicament for the therapeutic and/or prophylactic treatment of haemorrhage. The invention also provides a method for treating or preventing haemorrhage in a subject in need thereof, comprising administering to said subject effective dosage amount of an oxytocin formulation according to the invention. Still further, the invention provides a method for treating or preventing diabetes insipidus or vasodilatory shock in a subject in need thereof, comprising administering to said subject an effective dosage amount of a vasopressin formulation according to the invention.

In one aspect, the invention relates to a pH-buffered aqueous formulation comprising a therapeutically effective amount of oxytocin or an analogue thereof and at least one non-toxic biocompatible) source of divalent metal ions in a concentration of least 2 mM.

The term "oxytocin" as used herein includes both the original nonapeptide having the amino acid sequence as described above, as well as functional analogs desamino-oxytocin, carbetocin, 4-threonine-1-hydroxy-deaminooxytocin, 9-deamidooxytocin, 7-D-prolins-oxytocin and its deamino analog, (2,4-diisoleucine)-oxytocin, 1-desamino-1. monocarba-E12-Tyr (OMe)]-OT(dCOMOT), [Th⁴-Gly⁷]-oxytocin (TG-OT), the oxytocin agonist as described by Olson et al., (Peptides 12(1), 113-118, 1991), oxypressin and deamino-6-carba-oxytoxin (dC60). Further oxytoxin analogues (oxytocies) can for instance be found in MX 9 707 899, disclosing the oxytocin analogues (Mpa¹) oxytocin, (Mpa¹, D-Tyr(Et)², Thr⁴, Orn⁸) oxytocin, (Mpa¹, Ile²) oxytocin, (Mpa¹, Ala²) oxytocin, (Ile²) oxytocin, Gly-Leu⁴, Ile⁸) oxytocin, (D-Asn⁵) oxytocin, (D-Cys¹) oxytocin, (Gly¹) oxytocin, (Leu⁴, D-Arg⁹) oxytocin, (Mpa¹, Leu⁴, D-Arg⁸) oxytocin, (Arg⁸) oxytocin and (Ile^{e}) oxytocin.

In another embodiment, a formulation comprises vesopressin or an analogue thereof. As used herein, "vasopressin" is defined as including both the original nonapeptide having the amino acid sequence as described above, and vasopressin analogues desmopressin (1-desamino-8-D-arginine vasopressin or DDAVP, trade names Stimate®, Minirin® and Octostim®), felypressin, phenypressin, lypressin (also known as lysine vasopressin or LVP), ornipressin, terlipressin (trade name Glypressin®), pitressin (8-L-arginine vasopressin, NC-1900, AVP₄₋₉, desglycinamide-argmine⁸-vasopressin (DGAVP), d(CH₂)₆-Cys-d-Tyr(Et)-Arg-Val-Asn-Cys-Lys-Lys-ethylene diamine(TA-LVP), d(CH₂)₅-Tyr(Me) arginine vasopressin, 4-valine-8-D-arginine vasopressin (VDAVP), [His^{1.6}]AVP, and the vasopressin analogues disclosed in US 5,698,516. Further examples of vasopressin analogues can for instance be found in MX 9 707 899, disclosing the vasopressin analogues (Phe⁴, Arg⁸) vasopressin, (Mpa¹, Arg⁸) vasopressin, (Lys⁸) vasopressin, des-GlyNH₂. (Lys⁸) vasopressin, Gly-Gly-Gly-Lys⁸) vasopressin, (Mpa¹, D-Arg⁸) vasopressin, des-GlyNH₂-(Mpa¹, D-Arg⁸) vasopressin, (Mpa¹, Gly⁴, D-Arg⁸) vasopressin, (Mpa¹, Ala⁴, D-Arg⁸) vasopressin, (Mpa¹, Val⁴, D-Arg⁸) vasopressin, (Mpa¹, Ile⁴, D-Arg⁸) vasopressin, (Mpa¹, Leu⁴, D-Arg⁸) vasopressin and (Mpa¹, D-Gly⁸) vasopressin.

The source of the divalent mental ions may be one or more metal salts. Preferably, the source is a metal chloride salt. Any type of biocompatible metal chloride salt may be used as metal ion source. The divalent metal ions are preferably selected from the group consisting of Ca²⁺, Mg²⁺, Cu²⁺ and Zn²⁺ and are used at non-toxic concentrations and amounts. A preferred divalent metal ion is Ca²⁺. Useful metal chloride salts include CaCl₂, MgCl₂, CuCl₂ and ZinCl₂. Preferably, CaCl₂ and/or ZnCl₂ are used. As used herein, the concentration of at least 2 mM divalent metal ions refers to the total amount of divalent metal ions in case two or more distinct (sources of) divalent metal ions are used.

As shown herein below, the stabilizing effect of the combination of a buffer and at least one source of divalent metal ions at a concentration of at least 2 mM in an aqueous oxytocin formulation was determined by measuring the recovery of oxytocin (remaining oxytocin as % of initial amount), the amount of oxytocin-monomers (% of total remaining oxytocin) and / or the level of aggregation (aggregated oxytocin as % of total remaining oxytocin) upon prolonged storage at elevated (e.g. 55°C) temperatures. These parameters can be assessed by using any method for analysis deemed suitable by a person skilled in the art. Examples of suitable methods for analysis include, but are not limited to, reversed-phase high-performance liquid chromatography (RP-HPLC) and high-performance size exclusion chromatography (HP-SEC). Thus, suitable concentrations of divalent metal ions to achieve the stabilizing effect at elevated temperatures can be determined by those skilled in the art. They may vary, e.g. depending on the composition of the formulation or its intended application. In one embodiment, a formulation comprises oxytocin or vasopressin and at least 2 mM, preferably at least 5, more preferably at least 10 mM divalent metal ions and a buffer. In another embodiment, a pH-buffered formulation comprises divalent metal ions in a concentration of up to 150 mM, preferably up to 100 mM, more preferably up to 50 mM. For example, a formulation comprises divalent metal ions in a concentration between 5 and 150 mM, preferably between 10 and 100 mM, more preferably between 10 and 50 mM. In one embodiment, between 2 and 150 mM divalent metal ions, preferably 5-100 mM, such as 10, 20, 25, 30, 40 or 50 mM, and a buffer are used to stabilize an aqueous peptide formulation. In another embodiment, a divalent metal ion concentration in the range from 20 to 100 mM is used, like 30-100 mM, 40-80 mM or 50-70 mM. Other suitable ranges include between 2 and 50 mM, between 30 and 60 mM and between 40 and 70 mM divalent metal ions.

The stability of an aqueous oxytocin or vasopressin formulation is enhanced by addition of a combination of at least one source of divalent metal ions in a concentration of at least 2 mM and a buffer, to maintain a stable pH. Good stability was observed under slightly acidic pH. The invention therefore provides a formulation comprising oxytocin or vasopressin or an analogue thereof, at least one non-toxic source of divalent metal ions in a concentration of at least 2 mM and a buffer and having a pH between 3 and 6, preferably between 3 and 5, more preferably between 3.8 and 4.8.

Useful buffers include phosphate buffer, acetate buffer, aspartate buffer and citric acid (also known as citrate) buffer. In one embodiment the invention provides a formulation comprising oxytocin, vasopressin or an analogue thereof and a source of non-toxic metal ions, further comprising a buffer selected from the group consisting of phosphate buffer, acetate buffer, aspartate buffer and citric acid buffer. Buffer concentrations may vary according to specific circumstances. Typically, 2-200 mM buffer is used. Other useful ranges include 5-150 mM or 5-100 mM, such as 5-50 mM or 5-25 mM. In one embodiment, the invention provides a formulation comprising oxytocin or vasopressin, at least 2 mM divalent metal ions, preferably at least 5 mM divalent metal ions, more preferably at least 10 mM divalent metal ions and between 10 and 100 mM acetate buffer. For example, a formulation may comprise oxytocin, 10 mM Mg²⁺ and 10 mM acetate buffer. In another embodiment, the invention provides a formulation comprising oxytocin or vasopressin or an analogue thereof, between 5 and 60 mM divalent metal ions and/or between 10 and 50 mM aspartate buffer. For example, a formulation comprises oxytocin, 50 mM CaCl₂ and 50 mM aspartate buffer. Good results were observed using citric acid buffer, for example 2-100 mM or 5-50 mM citrate. Therefore, in a preferred embodiment the invention provides a formulation comprising oxytocin or vasopressin, e.g. in a concentration of up to 50 IU/ml, divalent metal ions, preferably up to 50 mM, and citric acid buffer.

In one embodiment, a formulation comprises between 5 and 60 IU/ml oxytocin, between 50 and 100 mM divalent metal ions and citric acid buffer while having a pH of between 4 and 5. In another embodiment, a formulation comprises between 30 and 200 IU/ml oxytocin or vasopressin, between 10 and 80 mM divalent metal ions and a buffer while having a pH of between 4 and 5. In a preferred embodiment, a formulation comprises between 5 and 100 IU/ml oxytocin, between 5 and 50 mM divalent metal ions and citric acid buffer while having a pH of 4.0 to 4.5. For example, a formulation may comprise 10 IU/ml oxytocin, 20 mM MgCl₂ and 10 mM citric acid buffer.

In another embodiment, a formulation comprises aspartate buffer and at least 2 mM Zn²⁺ ions, for instance provided by at least 2 mM ZnCl₂. A formulation may, as another example, comprise oxytocin, 20 mM NaCl, 10 mM CaCl₂ and citrate buffer. A formulation may, for another example, comprise oxytocin or vasopressin, acetate buffer, 5-50 mM MgCl₂ or 5-50 mM CaCl₂. In one embodiment, a formulation comprises oxytocin, a buffer and between 2 and 50 mM MgCl₂. In another embodiment, a formulation comprises oxytocin or vasopressin, a buffer and between 30 and 60 mM divalent metal ions, such as CaCl₂. In another embodiment, a formulation comprises oxytocin or vasopressin and between 60 and 80 mM divalent metal ions. For example, a formulation may comprise 70 mM ZnCl₂ and aspartate buffer. In yet another embodiment, a formulation comprises oxytocin, a buffer and between 30 and 100 mM divalent metal ions. In a preferred embodiment, a formulation comprises oxytocin or vasopressin, citrate buffer or aspartate buffer and between 5 and 50 mM divalent metal ions.

In another aspect, the invention provides a pH-buffered aqueous formulation comprising oxytocin, vasopressin or an analogues thereof as recited in claim 1 and a non-toxic source of chloride anions. The source of chloride ions can be a divalent chloride salt, preferably a divalent chloride salt. Suitable concentrations of chloride anions can be determined by those skilled in the art and may vary depending on the composition of the formulation or its intended application, In one embodiment, a formulation according to the invention comprises oxytocin, a buffer and at least 0.06 mM chloride anions, preferably at least 0.6 mM, more preferably at least 2 mM. A formulation may comprise oxytocin, a buffer and up to 150 mM chloride anions, preferably up to 100 mM chloride anions, more preferably up to 50 mM chloride anions, In one embodiment, a formulation comprises oxytocin and between 2 and 100 mM chloride preferably between 5 and 50 mM chloride anions. In another embodiment a formulation comprises between 10 and 60 mM chloride anions.

Good results were obtained in the presence of at least 2 mM CaCl₂, MgCl₂ or ZnCl₂ and citric acid buffer. In one embodiment, the invention therefore provides a formulation comprising oxytocin or vasopressin or an analogue thereof, and citrate buffer. Preferably, the formulation comprises citrate buffer and Ca²⁺, Mg²⁺ or Zn²⁺. For example, it contains between 2 and 60 mM CaCl₂ and/or between 5 and 200 citric acid buffer. In another embodiment, the formulation comprises between 50 and 100 mM MgCl₂, ZnCl₂ or CaCl₂ and/or between 6 and 50 mM citric acid buffer. For example, a formulation comprises oxytocin or vasopressin, e.g. in a concentration of up to 50 IU/ml, 50 mM MgCl₂ and 50 mM citric acid buffer. In yet another embodiment, a formulation comprises between 1 and 100 TU/ml oxytocin, between 30 and 80 mM MgCl₂, ZnCl₂ or CaCl₂ and/or between 30 and 60 may citric acid buffer. In a preferred embodiment, a formulation comprises between 10 and 100 IU/ml oxytocin or vasopressin, between 6 and 60 mM CaCl₂ and between 10 and 60 mM citric acid buffer. For example, a formulation may comprise oxytocin or vasopressin, e.g. in a concentration of up to 50 IU/ml, 10 mM CaCl₂ and 10 mM citric acid buffer.

Good stability was also observed in the presence of at least 2 mM Zn²⁺ and aspartate buffer. In one embodiment, the invention therefore provides a formulation comprising oxytocin or vasopressin or an analogue thereof as recited in claim 1 and aspartate buffer. Preferably, a formulation comprises oxytocin or vasopressin, aspartate buffer and Zn²⁺. For example, it contains between 2 and 60 mM ZnCl₂ and/or between 10 and 70 mM aspartate buffer. In another embodiment, the formulation comprises between 30 and 70 mM Zn²⁺ and/or between 10 and 50 mM aspartate buffer. For example, a formulation comprises oxytocin or vasopressin, e.g. in a concentration of up to 50 IU/ml, 40 mM ZnCl₂ and 20 mM aspartate buffer. In yet another embodiment, a formulation comprises between 1 and 100 IU/ml oxytocin or vasopressin, between 10 and 60 mM Zn²⁺ and/or between 30 and 60 mM aspartate buffer. In a preferred embodiment, a formulation comprises between 5 and 100 IU/ml oxytocin or vasopressin, between 5 and 50 mM Zn²⁺ and between 10 and 50 mM aspartate buffer. For example, a formulation comprises oxytocin or vasopressin, e.g. in a concentration of up to 80 IU/ml, 50 mM ZnCl₂ and 10 mM aspartate buffer.

As will be understood, a formulation as provided herein comprises a therapeutically effective amount of oxytocin or vasopressin or an analogue thereof as recited in claim 1 as exemplified above. In one aspect, the invention provides a formulations comprising at least 5 mIU/ml, more preferably at least 60 mIU/ml, more preferably at least 500 mIU/ml oxytocin or vasopressin or an analogue thereof as recited in claim 1. In another aspect, the invention provides a formulation comprising up to 1000 IU/ml, more preferably up to 500 IU/ml, more preferably up to 100 IU/ml oxytocin or vasopressin or an analogue thereof, as recited in claim 1 at least one non-toxic source of divalent metal ions in a concentration of at least 2 mM and a buffer. In one embodiment, a formulation comprises at least 500 mIU/ml oxytocin, a buffer and, preferably at least 2 mM, divalent metal ions such as Ca²⁺. In another embodiment, a formulation comprises between 5 and 300 IU/ml oxytocin, a buffer and, preferably between 2 and 50 mM, divalent metal ions. In another embodiment, a formulation comprises between 1 and 60 IU/ml oxytocin or vasopressin and, preferably between 10 and 90 mM, divalent metal ions such as Mg²⁺. In yet another embodiment, a formulation comprises between 10 and 200 IU/ml oxytocin, a buffer and, preferably between 50 and 80 mM, divalent metal ions. In a preferred embodiment, a formulation comprises between 5 and 100 IU/ml oxytocin or vasopressin, a buffer and, preferably between 5 and 50 mM, divalent metal ions such as CaCl₂. Also provided is a formulation comprising oxytocin or vasopressin or an analogue thereof in a concentration of between 5 and 100 IU/ml, between 6 and 50 mM Ca²⁺, between 10 and 50 mM citrate buffer and wherein the pH of said formulation is between 3.8 and 4.8. Another preferred formulation comprises oxytocin, vasopressin or an analogue thereof in a concentration of between 5 and 100 IU/mL between 5 and 50 mM Zn²⁺, between 10 and 50 mM aspartate buffer and wherein the pH of said formulation is between 3.8 and 4.8.

According to another object of the invention, the invention provides the use of a combination of a buffer and at least one non-toxic source of divalent metal ions and/or chloride anions in a concentration of at least 2 mM to stabilize an aqueous solution of oxytocin, vasopressin or an analogue thereof. The as recited in claim 15 source(s) and concentrations of these bivalent metal ions which may be used are described herein above. Preferably, the source is a metal salt. More preferably, the source is a metal chloride salt. For example, the metal chloride salt is selected from the group consisting of CaCl₂, MgCl₂, CuCl₂ and ZnCl₂. Preferably, the buffer is citrate buffer, acetate buffer or aspartate buffer. Preferred combinations include aspartate buffer plus Zn²⁺ ions; and citrate buffer plus Ca²⁺ and/or Mg²⁺ ions.

The invention also provides a pH-buffered aqueous formulation according to claim 1 for use as a medicament. Also provided is container, such as a package or spray container, comprising a formulation according to the invention. In view of the light sensitivity of oxytocin or vasopressin injectables, a package is preferably light resistant. Exemplary packages include vials, ampoules, plastic containers or a sprayer. The package may contain instructions for use.

According to another aspect of the invention, the invention provides the use of an aqueous formulation according to claim 1, for the manufacture of a medicament for the therapeutic and/or prophylactic treatment of haemorrhage, such as post-partum haemorrhage. Also provided is the use of aqueous formulation according to claim 1 for the manufacture of a medicament for the therapeutic and/or prophylactic treatment of diabetes insipidus or vasodilatory shock. These medicaments may be administered to a subject in need thereof by any means known to be suitable to those skilled in the art, including intravenous, subcutaneous, intramuscular, and mucosal administration.

A formulation may thus be formulated for administration via the intravenous, subcutaneous, intramuscular, and mucosal route. A formulation is preferably administered via the subcutaneous route, the mucosal route, or a combination thereof. The mucosal route may be exemplified by, but is not limited to, the pulmonary, nasal, sublingual or buccal route. Accordingly, in one embodiment the invention provides the use of formulations according to the invention, for the manufacture of a medicament formulated for subcutaneous or mucosal administration, or a combination thereof.

For intravenous, subcutaneous or intramuscular administration, the formulation may be provided as a sterile solution, suspension or an emulsion. The formulation may be applied by means of an injection or an infusion. For mucosal administration, the formulation may be provided as an aqueous spray and may be applied directly by means of a spray container or an inhalator. Alternatively, but not limited to, the formulation may be administered to the mucosa as an aqueous gel.

According to another aspect of the invention, the invention provides a method for treating or preventing haemorrhage in a subject in need thereof, comprising administering to said subject an effective dosage amount of an oxytocin formulation according to the invention. The haemorrhage may be post-partum haemorrhage. Also provided is a method for treating or preventing diabetes insipidus or vasodilatory shock in a subject in need thereof, comprising administering to said subject an effective dosage amount of a vasopressin formulation according to the invention. The subject may be living under (sub)tropical conditions. The effective dosage amount according to methods of the invention may be administered via the intravenous, subcutaneous or intramuscular route, the mucosal route, or a combination thereof. As mentioned supra, the mucosal route may be exemplified by, but is not limited to, the pulmonary, nasal, sublingual or buccal route.

Effective amounts of oxytocin or an analogue thereof are amounts that are sufficient to bring about an efficacious effect against symptoms associated with haemorrhage, and can be determined by those skilled in the art by routine experimentation. Effective amounts of vasopressin or an analogue thereof against diabetes insipidus or vasodilatory shock can be determined in a similar fashion. Furthermore, professional guidelines on the use of oxytocin or vasopressin in various indications exist and their use has been described in handbooks (e.g. AHFS Drug Information or Martindale, The Extra Pharmacopoeia).

In general, an effective dosage of oxytocin or an analogue thereof for the purposes of this invention is expected to comprise a bolus amount of between 2 and 10 IU. Thereafter, 5-10 IU or, in serious cases, up to 20 IU of oxytocin or an analogue thereof may be administered by infusion. In one aspect the invention provides a method for treating or preventing haemorrhage in a subject in need thereof, comprising administering to said subject a bolus amount of up to 2 IU of oxytocin or an analogue thereof, preferably up to 5 IU, more preferably up to 10 IU. In another aspect the invention provides a method for treating or preventing haemorrhage in a subject in need thereof, comprising administering to said subject a bolus amount of up to 2 IU, preferably up to 5 IU, more preferably up to 10 IU of oxytocin or an analogue thereof, further comprising an infusion of up to 20 IU, preferably up to 10 IU of oxytocin or an analogue thereof.

For vasopressin or an analogue thereof, a bolus dose of up to 20 IU may be administered intravenously in case of vasodilatory shock, or administered subcutaneously, intramuscularly or intranasally in case of diabetes insipidus. Thus, in another aspect the invention provides a method for treating or preventing diabetes insipidus or vasodilatory shock in a subject in need thereof, comprising administering to said subject a bolus amount of up to 20 IU of vasopressin or an analogue thereof, preferably up to 10 IU, more preferably up to 5 IU. In case of vasodilatory shock, a dosage of between 0.2 and 0.4 IU/min of vasopressin or an analogue thereof may be administered thereafter by infusion, preferably together with norepinephrine. According to FDA Guidelines, this dosage may be increased to 0.9 IU/min if necessary. In case of diabetes insipidus, a bolus dosage of 5 to 10 IU may be repeated two or three times daily as needed. When vasopressin is administered intranasally such as by spray, the dosage and interval between treatments must be determined for each subject.

The invention is exemplified by the following examples.

### LEGENDS TO THE FIGURES

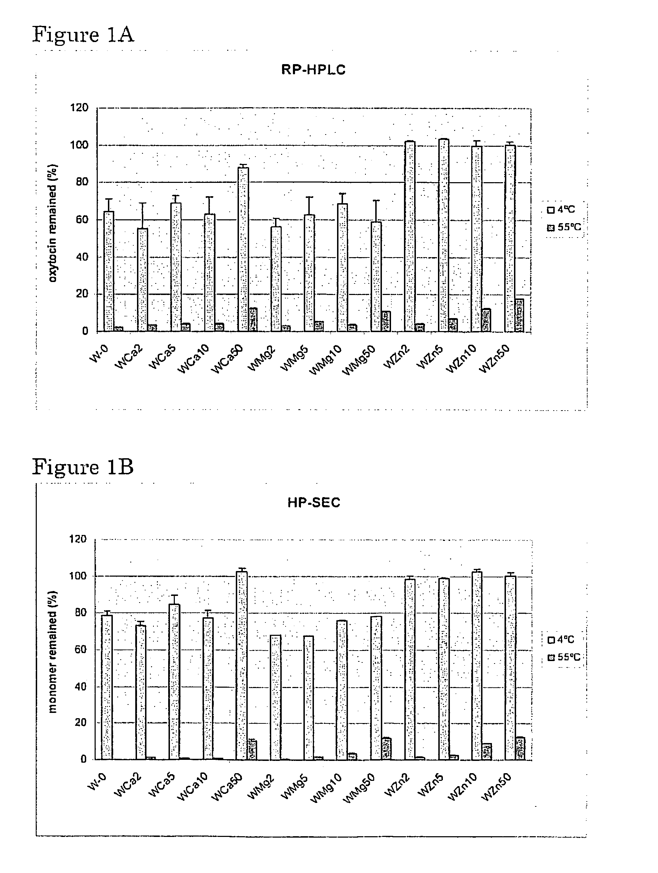

Figure 1: Recovery of oxytocin in the presence of divalent metal ions in non-buffered, pure water (W). The divalent metal ions (Ca²⁺, Mg²⁺ and Zn²⁺) were used in concentrations of 2, 5, 10 and 50 mM. The formulations were stored for 4 weeks at pH 4.5 and a temperature of 4 or 55 °C. Panel A: recovery determined by RP-HPLC. Panel B: oxytocin monomer recovery determined by HP-SEC. The results are depicted as averages of three independent measurements ± SD.

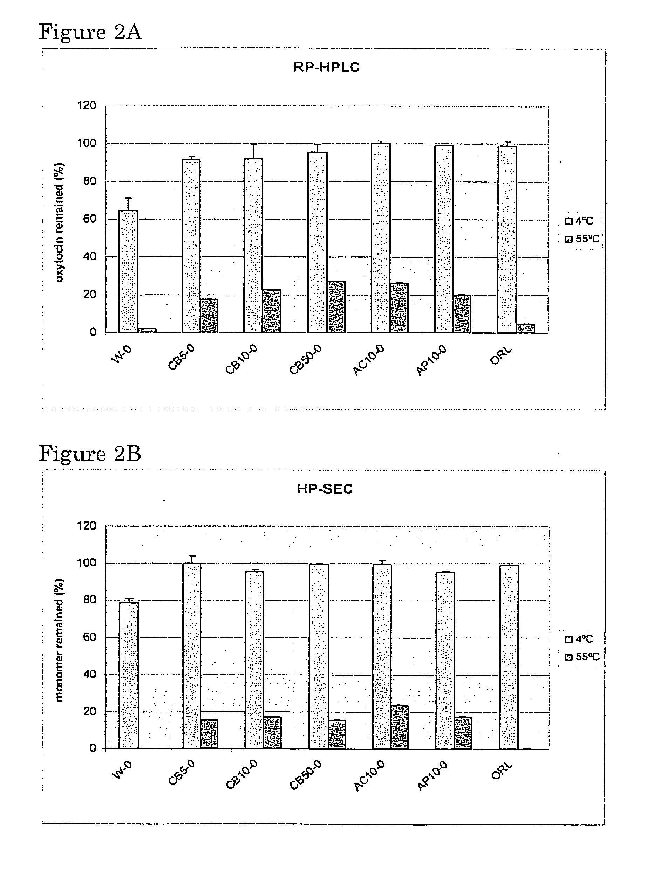

Figure 2: Recovery of oxytocin in pure water (W), with or without a buffer. Citrate buffer (CB) at a concentration of 5, 10 or 50 mM, acetate buffer (AC) at a concentration of 10 mM, aspartate buffer (AP) at a concentration of 10 mM or Ringer's lactate buffer (ORL) were used. The formulations contained no metal ions and were stored for 4 weeks at pH 4.5 or 6.4 (ORL) and at a temperature of 4 or 55 °C. Panel A: recovery determined by RP-HPLC. Panel B: oxytocin monomer recovery determined by HP-SEC. The results are depicted as averages of three independent measurements ± SD.

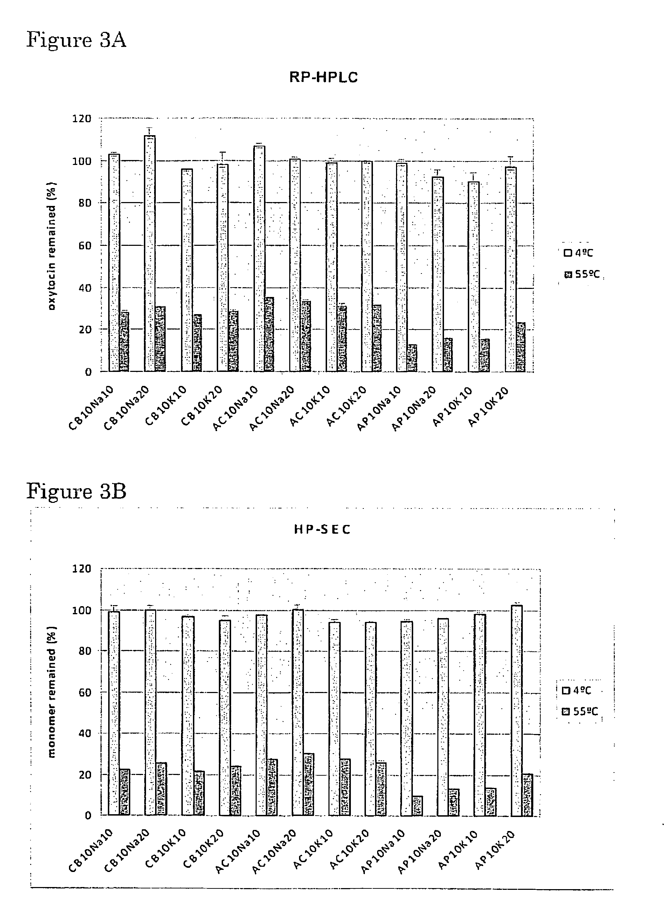

Figure 3: Recovery of oxytocin in the presence of buffer and monovalent metal ions. Citrate buffer (CB), acetate buffer (AC) and aspartate buffer (AP) were used at a concentration of 10 mM. Monovalent metal ions Na⁺ and K⁺ were used at concentrations of 10 or 20 mM. The formulations were stored for 4 weeks at pH4.5 and at a temperature of 4 or 55 °C. Panel A: recovery determined by RP-HPLC. Panel B: oxytocin monomer recovery determined by HP-SEC. The results are depicted as averages of three independent measurements ± SD.

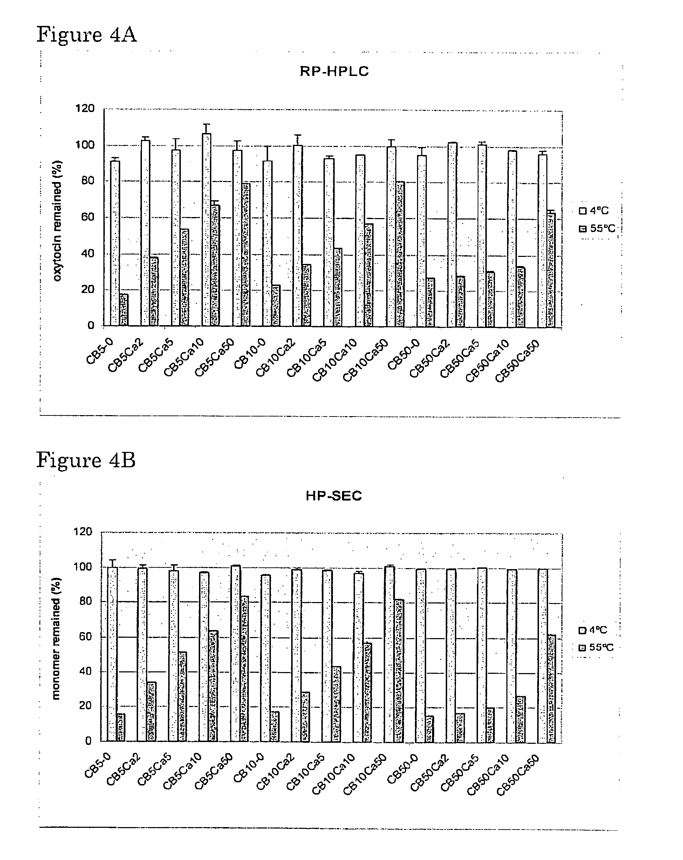

Figure 4: Recovery of oxytocin in citrate buffer in the absence or presence of Ca²⁺. Citrate buffer (CB) was used at concentrations of 5, 10 and 50 mM. Ca²⁺ was used at concentrations of 2, 5, 10 and 50 mM. All formulations were stored for 4 weeks at 4 or 55 °C and pH 4.5. Panel A: recovery determined by RP-HPLC. Panel B: oxytocin monomer recovery determined by HP-SEC. The results are depicted as averages of three independent measurements ± SD.

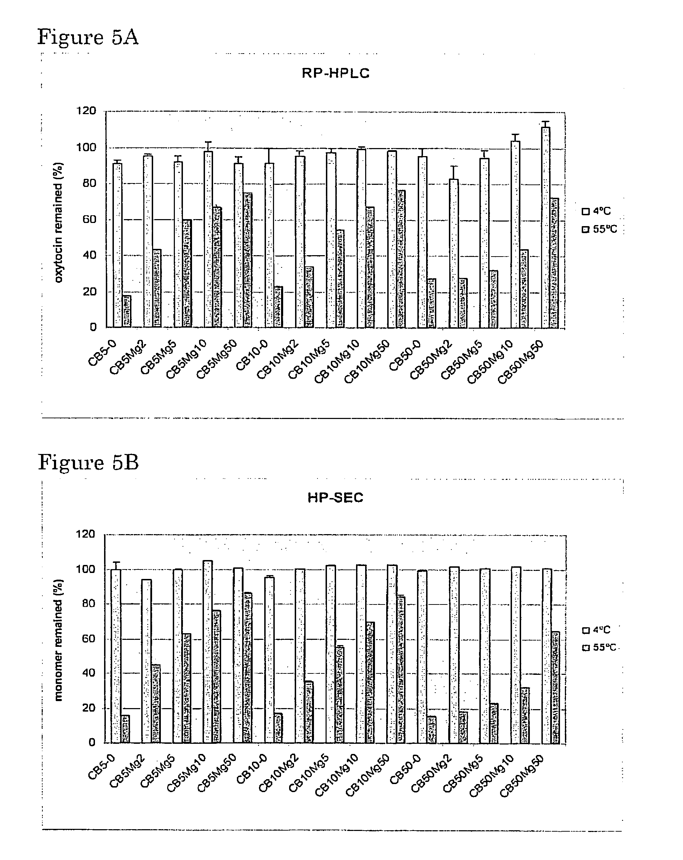

Figure 5: Recovery of oxytocin in citrate buffer in the absence or presence of Mg²⁺. Citrate buffer (CB) was used at concentrations of 5, 10 and 50 mM. Mg²⁺ was used at concentrations of 2, 5, 10 and 50 mM. All formulations were stored for 4 weeks at 4 or 55 °C and pH 4.5. Panel A: recovery determined by RP-HPLC. Panel B: oxytocin monomer recovery determined by HP-SEC. The results are depicted as averages of three independent measurements ± SD.

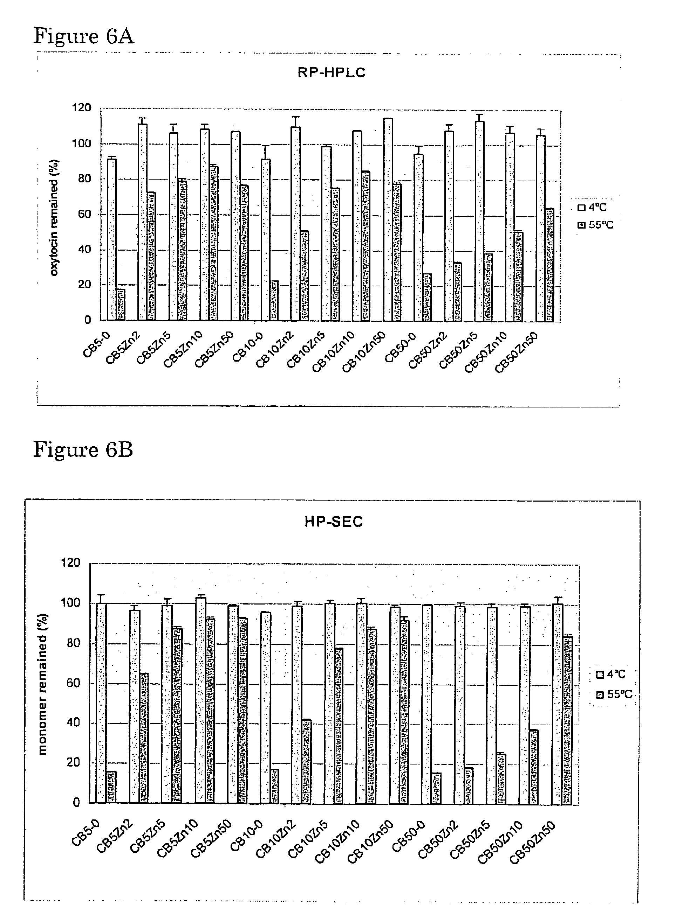

Figure 6: Recovery of oxytocin in citrate buffer in the absence or presence of Zn²⁺. Citrate buffer (CB) was used at concentrations of 5, 10 and 50 mM. Zn²⁺ was used at concentrations of 2, 5, 10 and 50 mM. All formulations were stored for 4 weeks at 4 or 55 °C and pH 4.5. Panel A: recovery determined by RP-HPLC. Panel B: oxytocin monomer recovery determined by HP-SEC. The results are depicted as averages of three independent measurements ± SD.

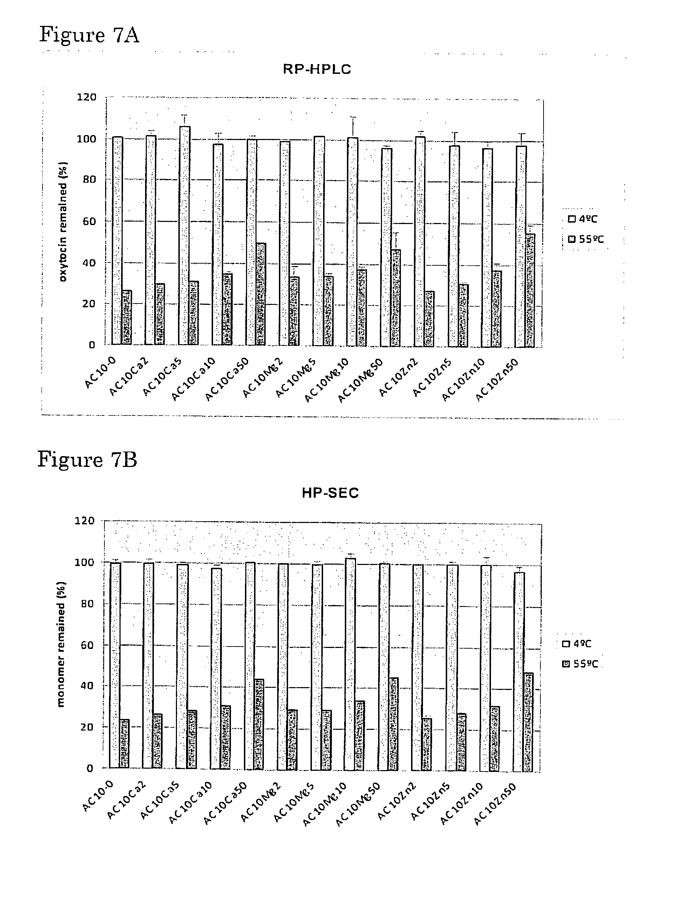

Figure 7: Oxytocin recovery in the presence of 10 mM acetate buffer (AC), with or without divalent metal ions. Divalent metal ions (Ca²⁺, Mg²⁺ and Zn²⁺) were used in concentrations of 0, 2, 5, 10 and 50 mM. Panel A: recovery after 4 weeks-storage at 4 or 55°C and pH 4.5, as determined by RP-HPLC. Panel B: oxytocin monomer recovery after 4 weeks storage at a temperature of 55°C and pH 4.5, as determined by HP-SEC. The results are depicted as averages of three independent measurements ± SD.

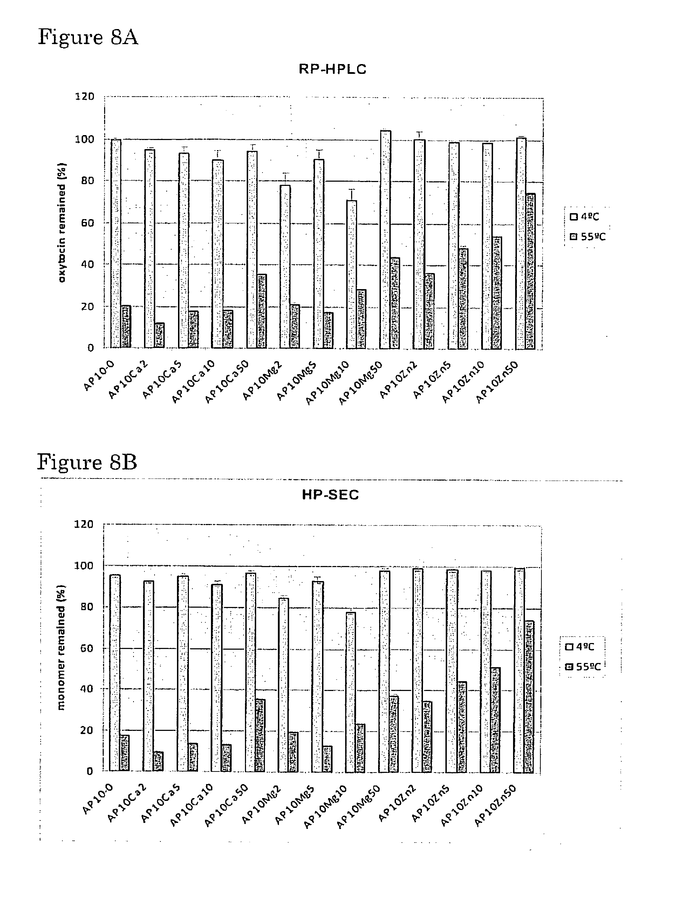

Figure 8: Oxytocin recovery in the presence of 10 mM aspartate buffer (AP), with or without divalent metal ions. Divalent metal ions (Ca²⁺, Mg²⁺ and Zn²⁺) were used in concentrations of 0, 2, 5, 10 and 50 mM. Panel A: recovery after 4 weeks-storage at 4 or 55 °C and pH 4.5, as determined by RP-HPLC. Panel B: oxytocin monomer recovery after 4 weeks storage at a temperature of 55 °C and pH 4.5, as determined by HP-SEC. The results are depicted as averages of three independent measurements ± SD.

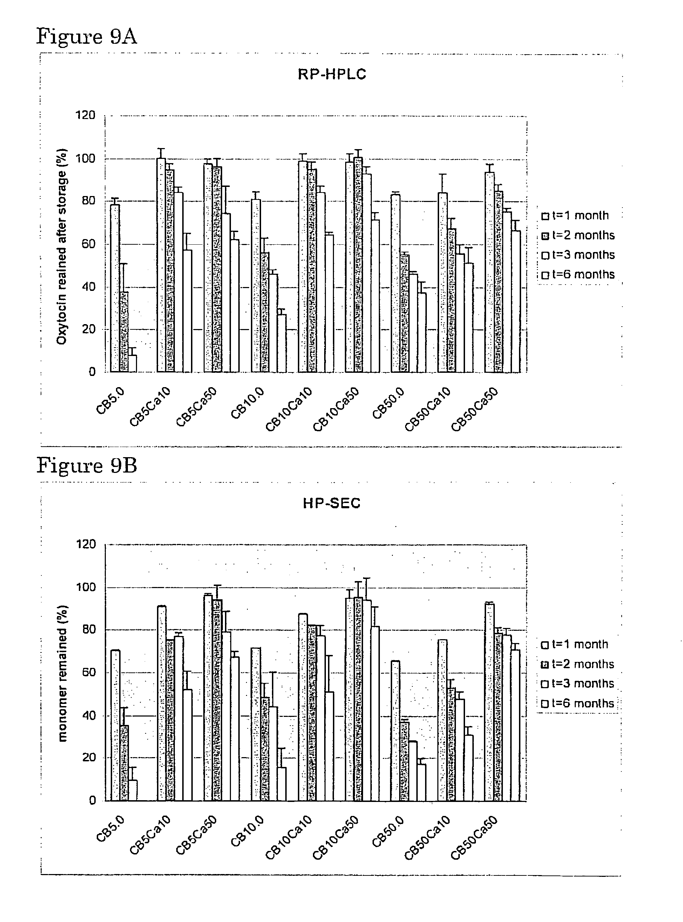

Figure 9: Recovery of oxytocin in 10 mM citrate buffer (CB) and 10 or 50 mM divalent metal ions (Ca²⁺). The formulations were stored at 40 °C and pH 4.5 for 1, 2, 3 or 6 months. Panel A: recovery determined by RP-HPLC. Panel B: oxytocin monomer recovery determined by HP-SEC. The results are depicted as averages of three independent measurements ± SD.

### EXPERIMENTAL SECTION

### Sample codes:

First character(s) refer to the type of buffer (or water)
W: water
CB: citrate buffer pH 4.5
AC: acetate buffer pH 4.5
AP: aspartate buffer pH 4.5
ORL: ringer lactate buffer
following digit(s) refer to buffer concentration in mM,
following character(s) refer to type of metal ion,
following digit(s) refer to metal ion concentration in mM.

All salts were chlorides (MClₓ)

E.g. CB5Mg10 means 5 mM citrate buffer pH 4.5 and 10 mM MgCl₂.

### EXAMPLE 1 (comparative)

This comparative example demonstrates the oxytocin-stabilizing effect of divalent metal ions. Divalent metal ions (Ca²⁺, Mg²⁺ and Zn²⁺) were added as metal chloride salts (MCl₂) to a non-buffered formulation comprising oxytocin and pure water (W). The divalent metal ions were used at concentrations of 0, 2, 5, 10 and 50 mM. Samples of the formulation were then stored for 4 weeks at pH 4.5, either at 4°C (reference control) or 55 °C (test). Thereafter, all samples were stored under cooled conditions (2-8 °C) before analysis by RP-HPLC and HP-SEC. Effects on stability were determined by measuring the recovery of oxytocin and the percentage of oxytocin-monomers. The results are presented in Figures 1A and 1B.

Figure 1A shows the recovery of oxytocin after 4 weeks-storage at 55 °C in non-buffered pure water (W) in the absence or presence of divalent metal ions. Oxytocin recovery at 4 °C was increased in the presence of Ca²⁺ or Zn²⁺. This effect was observed in a concentration-dependent manner. However, at 55 °C only poor stabilizing effects could be observed. Figure 1B shows the percentage of remaining oxytocin-monomers after 4 weeks-storage at 55 °C in non-buffered pure water (W) in the absence or presence of divalent metal ions. Similarly, loss of monomers was only prevented at 4 °C in the presence of Ca²⁺ or Zn²⁺. These results demonstrate a small stabilizing effect of divalent metal ions on an aqueous oxytocin formulation.

### EXAMPLE 2 (comparative)

Citrate buffer (CB) at concentrations of 0, 5, 10 or 50 mM, acetate buffer (AC) at concentrations of 10 mM, aspartate buffer (AP) at concentrations of 10 mM or Ringer's lactate buffer (ORL) were added to formulations comprising oxytocin in pure water (W). Ringer lactate used was Baxter Viavlo 500 mL WE2323 Ringer lactaat, solution for iv infusion. Lot number 09B04E1P exp. date 2011. The pH of the preparation was 6.4.

### Composition per 1000 mL:

| | | | | |
|---|---|---|---|---|
| Sodium chloride | 6 | g | Sodium | 131 mmol |
| Potassium chloride | 0,4 | g | Potassium | 5 mmol |
| Calcium chloride dihydrate | 0,27 | g | Calcium 2 mmol, Chloride | 111 mmol |
| Sodium lactate | 3,2 | g | Bicarbonate (as lactate) | 29 mmol |
| Water for injections qs | | | | |

All formulations were free of divalent metal ions (except for ORL) and had a final oxytocin-concentration of 0.1 mg/ml and a pH of either 4.5 (CB, AC and AP) or 6.4 (ORL). Samples of each formulation were stored for 4 weeks at 4 °C (control) or 55 °C (test). Thereafter, all samples were stored under cooled conditions (2-8 °C) before analysis by RP-HPLC and HP-SEC. Effects on stability were determined by measuring the recovery of oxytocin and the percentage of oxytocin-monomers. The results are presented in Figures 2A and 2B.

Figure 2A shows the recovery of oxytocin after 4 weeks-storage at 55 °C in pure water (W), with or without addition of a buffer and in the absence of divalent metal ions. At 4 °C, oxytocin recovery was substantially increased in the presence of a buffer. At 55°C a small increase in oxytocin stability could be observed in the presence of citrate buffer, acetate buffer or aspartate buffer, but not in the presence of Ringer's lactate buffer. These findings were reflected by the percentage of remaining oxytocin-monomers, as depicted in figure 2B. These results demonstrate a small stabilizing effect of citrate buffer, acetate buffer or aspartate buffer, but not Ringer's lactate buffer, on an aqueous oxytocin formulation at tropical conditions.

### EXAMPLE 3 (comparative)

The thermal stability of oxytocin in the presence of combinations of a buffer and monovalent metal ions was investigated. Citrate buffer (CB), acetate buffer (AC) or aspartate buffer (AP) was used at a concentration of 10 mM. Monovalent metal ions Na⁺ and K⁺ were added in the form of their chloride salt (NaCl and KCl) and used in final concentrations of 10 and 20 mM. All formulations had a final oxytocin-concentration of 0.1 mg/ml and a pH of 4.5. Samples of each formulation were stored for 4 weeks at 4 °C (control) or 55 °C (test). Thereafter, all samples were stored under cooled conditions (2-8 °C) before analysis by RP-HPLC and HP-SEC. Effects on stability were determined by measuring the recovery of oxytocin and the percentage of oxytocin-monomers. The results are presented in Figures 3A and 3B.

In Figure 3A, minor effects on the thermal stability of oxytocin stored at 55 °C can be observed. Although oxytocin stability was slightly increased compared to the presence of buffers alone (see Figures 2A and 2B), still no more than up to approximately 35% of the original quantity of oxytocin was recovered (10 mM acetate buffer & 10 mM Na⁺). Of this remaining part, only around 30% had remained in its monomeric form (Figure 3B). These results clearly demonstrate that the presence of a combination of a buffer and monovalent metal ions is inadequate to stabilize oxytocin under tropical conditions.

### EXAMPLE 4

This example demonstrates the strong thermal stability of oxytocin formulations comprising citrate buffer and divalent metal ions. Citrate buffer (CB) was used at concentrations of 5, 10 or 50 mM. Divalent metal ions (Ca²⁺, Mg²⁺ and Zn²⁺) were added in the form of their chloride salt (CaCl₂, MgCl₂ and ZnCl₂) and used in final concentrations of 0, 2, 5, 10 or 50 mM. All formulations had a final oxytocin-concentration of 0.1 mg/ml and a pH of 4.5. Samples of each formulation were stored for 4 weeks at 4 °C (control) or 55 °C (test). Thereafter, all samples were stored under cooled conditions (2-8 °C) before analysis by RP-HPLC and HP-SEC. Effects on stability were determined by measuring the recovery of oxytocin and the percentage of oxytocin-monomers. The results are presented in Figures 4A and 4B (citrate buffer with Ca²⁺), 5A and 5B (citrate buffer with Mg²⁺) and 6A and 6B (citrate buffer with Zn²⁺).

Figures 4A and 4B show the recovery of oxytocin and the remaining percentage of oxytocin monomers after 4 weeks-storage at 55 °C in citrate buffer (CB) in the absence or presence of Ca²⁺. Both oxytocin recovery and the percentage of oxytocin monomers were increased up to 80% in the presence of Ca²⁺ in a concentration-dependent manner. These results demonstrate the stabilizing effect of citrate buffer and Ca²⁺ on an aqueous oxytocin formulation. Largest increases in stability were observed in the presence of 5 or 10 mM citrate buffer and 50 mM Ca²⁺. At 50 mM citrate buffer, oxytocin stability is only increased together with 50 mM Ca²⁺. At 100 mM Ca²⁺, oxytocin precipitates were observed.

Similar effects can be observed in Figures 5A and 5B, showing the recovery of oxytocin and the remaining percentage of oxytocin monomers after 4 weeks-storage at 55 °C in citrate buffer (CB) in the absence or presence of Mg²⁺. Again, largest increases in stability were observed in the presence of 5 or 10 mM citrate buffer and 50 mM Mg²⁺. At 50 mM citrate buffer, oxytocin stability was also only increased at 50 mM Mg²⁺.

Figures 6A and 6B show the recovery of oxytocin and the remaining percentage of oxytocin monomers after 4 weeks-storage at 55 °C in citrate buffer (CB) in the absence or presence of Zn²⁺. Both oxytocin recovery and the percentage of remaining oxytocin monomers were increased up to 90% in the presence of Zn²⁺ in a concentration-dependent manner. However, large increases in stability were already observed in the presence of only 2 or 5 mM Zn²⁺. This suggests that combinations of citrate buffer and Zn²⁺ may exert even stronger effects on oxytocin stability than combinations of citrate buffer and Ca²⁺ or Mg²⁺. At 50 mM citrate buffer, oxytocin stability was only increased at 50 mM Zn²⁺.

### EXAMPLE 5

This example demonstrates a small increase in thermal stability of oxytocin formulations comprising 10 mM acetate buffer (AC) and divalent metal ions. Divalent metal ions (Ca²⁺, Mag²⁺ and Zn²⁺) were added in the form of their chloride salt (CaCl₂, MgCl₂ and ZnCl₂) and used in final concentrations of 0, 2, 5, 10 or 50 mM. All formulations had a final oxytocin-concentration of 0.1 mg/ml and a pH of 4.5. Samples of each formulation were stored for 4 weeks at 4 °C (control) or 55 °C (test). Thereafter, all samples were stored under cooled conditions (2-8 °C) before analysis by RP-HPLC and HP-SEC. Effects on stability were determined by measuring the recovery of oxytocin and the percentage of oxytocin-monomers. The results are presented in Figures 7A and 7B.

Figure 7A shows the remaining percentage of oxytocin after 4 weeks-storage at 55 °C in 10 mM acetate buffer (AC) in the absence or presence of divalent metal ions. Small increases in the remaining percentage of oxytocin could be observed in the presence of 50 mM Ca²⁺, 50 mM Mg²⁺ or 50 mM Zn²⁺. These effects can also be observed in Figure 7B. These results demonstrate a stability-enhancing effect of acetate buffer and divalent metal ions on aqueous oxytocin formulations.

### EXAMPLE 6

This example exemplifies the oxytocin-stabilizing effect of Zn²⁺ in combination with aspartate buffer (AP). Aspartate buffer was added in a concentration of 10 mM to formulations comprising oxytocin and 0, 2, 5, 10 or 50 mM CaCl₂, MgCl₂ or ZnCl₂. All formulations had a final oxytocin-concentration of 0.1 mg/ml and a pH of 4.5. Samples of each formulation were stored for 4 weeks at 4 °C (control) or 55 °C (test). Thereafter, all samples were stored under cooled conditions (2-8 °C) before analysis by RP-HPLC and HP-SEC. Effects on stability were determined by measuring the recovery of oxytocin and the percentage of oxytocin-monomers.The results are presented in Figures 8A and 8B, demonstrating a strong concentration-dependent stabilizing effect of Zn²⁺ and aspartate buffer. Up to 75% oxytocin remained after 4 weeks storage at 55 °C and pH 4.5. When aspartate buffer was used with Ca²⁺ or Mg²⁺, only smaller effects could be observed.

### EXAMPLE 7

A long-term stability study was performed to assess the thermal stability of pH-buffered oxytocin formulations comprising divalent metal ions over prolonged periods. Citrate buffer (CB) was added in concentrations of 5, 10 or 50 mM to formulations comprising 0.1 mg/ml oxytocin and 0, 10 or 50 mM Ca²⁺ (CaCl₂). The final formulations had a pH of 4.5. Samples of each formulation were stored for 1, 2, 3 or 6 months at 40 °C. Thereafter, all samples were stored under cooled conditions (2-8 °C) before analysis by RP-HPLC and HP-SEC. Effects on stability were determined by measuring the recovery of oxytocin and the percentage of oxytocin-monomers. The results of this study are presented in Figures 9A and 9B.

Figure 9A shows the recovery of oxytocin in the presence of different concentrations of CaCl₂ and 5 or 10 mM citrate buffer over time. In the presence of citrate buffer (CB) and Ca²⁺, oxytocin recovery is increased at every time point when compared to the presence of citric acid alone. The strong stability-increasing effect of citrate buffer and Ca²⁺ can be observed in a concentration-dependent manner. Although this effect decreases gradually over time, it remains quite powerful. For example, even after 6 months of storage at 40 °C still around 70% of the original amount of oxytocin in a formulation is preserved when using 10 mM citrate buffer and 50 mM Ca²⁺. As can be observed in Figure 9B, more than 80% of that portion has remained in its monomeric form. These results clearly demonstrate the stabilizing effects of combinations of a buffer and at least 2 mM divalent metal ions on oxytocin.

## Claims

1. An aqueous formulation comprising a therapeutically effective amount of oxytocin, desamino-oxytocin, carbetocin, 4-threonine-1-hydroxy-deaminooxytocin, 9-deamidooxytocin, 7-D-proline-oxytocin or its deamino analog, (2,4-diisoleucine)-oxytocin, 1-desamino-1-monocarba-E12-Tyr (OMe)]-OT(dCOMOT), [Thr⁴-Gly⁷]-oxytocin (TG-OT), oxypressin, deamino-6-carba-oxytoxin (dC60), (Mpa¹) oxytocin, (Mpa¹, D-Tyr(Et)², Thr⁴, Orn⁸) oxytocin, (Mpa¹, Ile²) oxytocin, (Mpa¹, Ala²) oxytocin, (Ile²) oxytocin, Gly-(Leu⁴, Ile⁸) oxytocin, (D-Asn⁵) oxytocin, (D-Cys¹) oxytocin, (Gly⁴) oxytocin, (Leu⁴, D-Arg⁸) oxytocin, (Mpa¹, Leu⁴, D-Arg⁸) oxytocin, (Arg⁸) oxytocin, (Ile⁸) oxytocin, ergomotrine, methylergometrine, vasopressin, desmopressin (1-desamino-8-D-arginine vasopressin or DDAVP), felypressin, phenypressin, lypressin (also known as lysine vasopressin or LVP), ornipressin, terlipressin, pitressin (8-L-arginine vasopressin, NC-1900, AVP₄₋₉, desglycinamide-arginine⁸-vasopressin (DGAVP), d(CH₂)₅₋Cys-d-Tyr(Et)-Arg-Val-Asn-Cys-Lys-Lys-ethylene diamine (TA-LVP), d(CH₂)₅-Tyr(Me) arginine vasopressin, 4-valine-8-D-arginine vasopressin (VDAVP), [His^{1,6}]AVP, (Phe⁴, Arg⁸) vasopressin, (Mpa¹, Arg⁸) vasopressin, (Lys⁸) vasopressin, des-GlyNH₂-(Lys8) vasopressin, Gly-Gly-Gly-(Lys⁸) vasopressin, (Mpa¹, D-Arg⁸) Vasopressin, des-GlyNH₂-(Mpa¹, D-Arg⁸) vasopressin, (Mpa¹, Gly⁴, D-Arg⁸) vasopressin, (Mpa¹, Ala⁴, D-Arg⁸) vasopressin, (Mpa¹, Val⁴, D-Arg⁸) vasopressin, (Mpa¹, Ile⁴, D-Arg⁸) vasopressin, (Mpa¹, Leu⁴, D-Arg⁸) vasopressin or (Mpa¹, D-Gly⁸) vasopressin, a buffer and at least one non-toxic source of divalent metal ions in a concentration of at least 2 mM.

2. The formulation according to claim 1, comprising divalent metal ions in a concentration between 5 and 150 mM.

3. The formulation according to claim 1 or 2 wherein said source of divalent metal ions is a metal salt.

4. The formulation according to any one of claims 1 to 3, comprising a buffer selected from the group consisting of citrate buffer, acetate buffer, phosphate buffer and aspartate buffer.

5. The formulation according to anyone of claims 1 to 4, wherein said divalent metal ions are selected from the group consisting of Ca²⁺, Mg²⁺, Cu²⁺ and Zn²⁺.

6. The formulation according to any one of claims 1 to 5, comprising citrate buffer and one or more of Ca²⁺, Mg²⁺ and Zn²⁺.

7. The formulation according to any one of claims 1 to 5, comprising aspartate buffer and Zn²⁺.

8. The formulation according to any one of claims 1 to 7, wherein said formulation comprises oxytocin, desamino-oxytocin, carbetocin, 4-threonine-1-hydroxy-deaminooxytocin, 9-deamidooxytocin, 7-D-proline-oxytocin or its deamino analog, (2,4-diisoleucine)-oxytocin, 1-desamino-1-monocarba-E12-Tyr (OMe)]-OT(dCOMOT), [Thr⁴-Gly⁷]-oxytocin (TG-OT), oxypressin, deamino-6-carba-oxytoxin (dC60), (Mpa¹) oxytocin, (MPa¹, D-Tyr(Et)², Thr⁴, Orn⁸) oxytocin, (MPa¹, Ile²) oxytocin, (Mpa¹, Ala²) oxytocin, (Ile²) oxytocin, Gly-(Leu⁴, Ile⁸) oxytocin, (D-Asn⁵) oxytocin, (D-Cys¹) oxytocin, (Gly⁴) oxytocin, (Leu⁴, D-Arg⁸) oxytocin, (Mpa¹, Leu⁴, D-Arg⁸) oxytocin, (Arg⁸) oxytocin or (Ile⁸) oxytocin, ergometrine, methylergometrine.

9. The formulation according to any one of claims 1 to 7, wherein said formulation comprises vasopressin, desmopressin (1-desamino-8-D-arginine vasopressin or DDAVP), felypressin, phenypressin, lypressin (also known as lysine vasopressin or LVP), ornipressin, terlipressin, pitressin (8-L-arginine vasopressin, NC-1900, AVP₄₋₉, desglycinamide-arginine⁸-vasopressin (DGAVP), d(CH₂)₅-Cys-d-Tyr(Et)-Arg-Val-Asn-Cys-Lys-Lys-ethylene diamine (TA-LVP), d(CH₂)₅-Tyr(Me) arginine vasopressin, 4-valine-8-D-arginine vasopressin (VDAVP), [His^{1,6}]AVP, (Phe⁴, Arg⁸) vasopressin, (Mpa¹, Arg⁸) vasopressin, (Lys⁸) vasopressin, des-GlyNH₂-(Lys⁸) vasopressin, Gly-Gly-Gly-(Lys⁸) vasopressin, (Mpa¹, D-Arg⁸) vasopressin, des-GlyNH₂-(Mpa¹, D-Arg⁸) vasopressin, (Mpa¹, Gly⁴, D-Arg⁸) vasopressin, (Mpa¹, Ala⁴, D-Arg⁸) vasopressin, (Mpa¹, Val⁴, D-Arg⁸) vasopressin, (Mpa¹, Ile⁴, D-Arg⁸) vasopressin, (Mpa¹, Lieu⁴, D-Arg⁸) vasopressin or (Mpa¹, D-Gly⁸) vasopressin.

10. The formulation according to any one of claims 1 to 9 for use as a medicament.

11. A container comprising a formulation according to any one of claims 1 to 10 and instructions for use.

12. The formulation according to claim 8 for use in the therapeutic and/or prophylactic treatment of haemorrhage, such as post-partum haemorrhage.

13. The formulation according to claim 9 for use in the therapeutic and/or prophylactic treatment of diabetes insipidus or vasodilatory shock.

14. The formulation for use according to claim 12 or 13, wherein said medicament is for a treatment by administration via the intravenous, subcutaneous or intramuscular route, the mucosal route, or a combination thereof.

15. Use of a combination of (i) a buffer, and (ii) at least one non-toxic source of divalent metal ions in a concentration of at least 2 mM to stabilize an aqueous formulation of oxytocin, desamino-oxytocin, carbetocin, 4-threonine-1-hydroxy-deaminooxytocin, 9-deamidooxytocin, 7-D-proline-oxytocin or its deamino analog, (2,4-diisoleucine)-oxytocin, 1-desamino-1-monocarba-E12-Tyr (OMe)]-OT(dCOMOT), [Thr⁴-Gly⁷]-oxytocin (TG-OT), oxypressin, deamino-6-carba-oxytoxin (dC60), (Mpa¹) oxytocin, (Mpa¹, D-Tyr(Et)², Thr⁴, Orn⁸) oxytocin, (Mpa¹, Ile²) oxytocin, (Mpa¹, Ala²) oxytocin, (Ile²) oxytocin, Gly-(Leu⁴, Ile⁸) oxytocin, (D-Asn⁵) oxytocin, (D-Cys¹) oxytocin, (Gly⁴) oxytocin, (Leu⁴, D-Arg⁸) oxytocin, (Mpa¹, Leu⁴, D-Arg⁸) oxytocin, (Arg⁸) oxytocin, (Ile⁸) oxytocin, ergometrine, methylergometrine, vasopressin desmopressin (1-dasamino-8-D-arginine vasopressin or DDAVP), felypressin, phenypressin, lypressin (also known as lysine vasopressin or LVP), ornipressin, terlipressin, pitressin (8-L-arginine vasopressin, NC-1900, AVP₄₋₉, desglycinamide-arginine⁸-vasopressin (DGAVP), d(CH₂)₅-Cys-d-Tyr(Et)-Arg-Val-Asn-Cys-Lys-Lys-ethylene diamine (TA-LVP), d(CH₂)₅-Tyr(Me) arginine vasopressin, 4-valine-8-D-arginine vasopressin (VDAVP), [His^{1,6}]AVP, (Phe⁴, Arg⁸) vasopressin, (Mpa¹, Arg⁸) vasopressin, (Lys⁸) vasopressin, des-GlyNH₂-(Lys⁸) vasopressin, Gly-Gly-Gly-(Lys⁸) vasopressin, (Mpa¹, D-Arg⁸) vasopressin, des-GlyNH₂-(Mpa¹, D-Arg⁸) vasopressin, (MPa¹, Gly⁴, D-Arg⁸) vasopressin, (Mpa¹, Ala⁴, D-Arg⁸) vasopressin, (Mpa¹, Val⁴, D-Arg⁸) vasopressin, (Mpa¹, Ile⁴, D-Arg⁸) vasopressin, (Mpa¹, Leu⁴, D-Arg⁸) vasopressin or (Mpa¹, D-Gly⁸) vasopressin,

## Patentansprüche

1. Wässrige Formulierung, umfassend eine therapeutisch wirksame Menge an Oxytocin, Desamino-Oxytocin, Carbetocin, 4-Threonin-1-Hydroxy-Deaminooxytocin, 9-Deamidooxytocin, 7-D-Prolin-Oxytocin oder sein Deamino-Analogon, (2,4-Düsoleucin)-Oxytocin, 1-Desamino-1-Monocarba-E12-[Tyr(OMe)]-OT(dCOMOT), [Thr⁴-Gly⁷]-Oxytocin (TG-OT), Oxypressin, Deamino-6-Carba-Oxytoxin (dC60), (Mpa¹) Oxytocin, (Mpa¹, D-Tyr(Et)², Thr⁴, Orn⁸) Oxytocin, (Mpa¹,Ile²) Oxytocin, (Mpa¹, Ala²) Oxytocin, (Ile²) Oxytocin, Gly-(Leu⁴, Ile⁸) Oxytocin, (D-Asn⁵) Oxytocin, (D-Cys¹) Oxytocin, (Gly⁴) Oxytocin, (Leu⁴, D-Arg⁸) Oxytocin, (Mpa¹, Leu⁴, D-Arg⁸) Oxytocin, (Arg⁸) Oxytocin, (Ile⁸) Oxytocin, Ergometrin, Methylergometrin, Vasopressin, Desmopressin (1-Desamino-8-D-Arginin-Vasopressin oder DDAVP), Felypressin, Phenypressin, Lypressin (auch bekannt als Lysin-Vasopressin oder LVP), Ornipressin, Terlipressin, Pitressin (8-L-Arginin-Vasopressin, NC-1900, AVP₄₋₉), Desglycinamid-Arginin⁸-Vasopressin (DGAVP), d(CH₂)₅-Cys-d-Tyr(Et)-Arg-Val-Asn-Cys-Lys-Lys-Ethylendiamin (TA-LVP), d(CH₂)₅-Tyr(Me)-Arginin-Vasopressin, 4-Valin-8-D-Arginin-Vasopressin (VDAVP), [His^{1,6}]AVP, (Phe⁴, Arg⁸) Vasopressin, (Mpa¹, Arg⁸) Vasopressin, (Lys⁸) Vasopressin, Des-GlyNH₂-(Lys⁸) Vasopressin, Gly-Gly-Gly-(Lys⁸) Vasopressin, (Mpa¹, D-Arg⁸) Vasopressin, Des-GlyNH₂-(Mpa¹, D-Arg⁸) Vasopressin, (Mpa¹, Gly⁴, D-Arg⁸) Vasopressin, (Mpa¹, Ala⁴, D-Arg⁸) Vasopressin, (Mpa¹, Val⁴, D-Arg⁸) Vasopressin, (Mpa¹, Ile⁴, D-Arg⁸) Vasopressin, (Mpa¹, Leu⁴, D-Arg⁸) Vasopressin oder (Mpa¹, D-Gly⁸) Vasopressin, einen Puffer und mindestens eine nicht toxische Quelle von zweiwertigen Metallionen in einer Konzentration von mindestens 2 mM.

2. Formulierung nach Anspruch 1, umfassend zweiwertige Metallionen in einer Konzentration zwischen 5 und 150 mM.

3. Formulierung nach Anspruch 1 oder 2, wobei die Quelle der zweiwertigen Metallionen ein Metallsalz ist.

4. Formulierung nach einem der Ansprüche 1 bis 3, umfassend einen Puffer, ausgewählt aus der Gruppe bestehend aus Citratpuffer, Acetatpuffer, Phosphatpuffer und Aspartatpuffer.

5. Formulierung nach einem der Ansprüche 1 bis 4, wobei die zweiwertigen Metallionen ausgewählt sind aus der Gruppe bestehend aus Ca²⁺, Mg²⁺, Cu²⁺ und Zn²⁺.

6. Formulierung nach einem der Ansprüche 1 bis 5, umfassend Citratpuffer und ein oder mehrere von Ca²⁺, Mg²⁺ und Zn²⁺.

7. Formulierung nach einem der Ansprüche 1 bis 5, umfassend Aspartatpuffer und Zn²⁺.

8. Formulierung nach einem der Ansprüche 1 bis 7, wobei die Formulierung Oxytocin, Desamino-Oxytocin, Carbetocin, 4-Threonin-1-Hydroxy-Deaminooxytocin, 9-Deamidooxytocin, 7-D-Prolin-Oxytocin oder sein Deamino-Analogon, (2,4-Düsoleucin)-Oxytocin, 1-Desamino-1-Monocarba-E12-[Tyr(OMe)]-OT(dCOMOT), [Thr⁴-Gly⁷]-Oxytocin (TG-OT), Oxypressin, Deamino-6-Carba-Oxytoxin (dC60), (Mpa¹) Oxytocin, (Mpa¹, D-Tyr(Et)², Thr⁴, Orn⁸) Oxytocin, (Mpa¹, Ile²) Oxytocin, (Mpa¹, Ala²) Oxytocin, (Ile²) Oxytocin, Gly-(Leu⁴, Ile⁸) Oxytocin, (D-Asn⁵) Oxytocin, (D-Cys¹) Oxytocin, (Gly⁴) Oxytocin, (Leu⁴, D-Arg⁸) Oxytocin, (Mpa¹, Leu⁴, D-Arg⁸) Oxytocin, (Arg⁸) Oxytocin oder (Ile⁸) Oxytocin, Ergometrin, Methylergometrin umfasst.

9. Formulierung nach einem der Ansprüche 1 bis 7, wobei die Formulierung Vasopressin, Desmopressin (1-Desamino-8-D-Arginin-Vasopressin oder DDAVP), Felypressin, Phenypressin, Lypressin (auch bekannt als Lysin-Vasopressin oder LVP), Ornipressin, Terlipressin, Pitressin (8-L-Arginin-Vasopressin, NC-1900, AVP₄₋₉), Desglycinamid-Arginin⁸-Vasopressin (DGAVP), d(CH₂)₅-Cys-d-Tyr(Et)-Arg-Val-Asn-Cys-Lys-Lys-Ethylendiamin (TA-LVP), d(CH₂)₅-Tyr(Me)-Arginin-Vasopressin, 4-Valin-8-D-Arginin-Vasopressin (VDAVP), [His^{1,6}]AVP, (Phe⁴, Arg⁸) Vasopressin, (Mpa¹, Arg⁸) Vasopressin, (Lys⁸) Vasopressin, Des-GlyNH₂-(Lys⁸) Vasopressin, Gly-Gly-Gly-(Lys⁸) Vasopressin, (Mpa¹, D-Arg⁸) Vasopressin, Des-GlyNH₂-(Mpa¹, D-Arg⁸) Vasopressin, (Mpa¹, Gly⁴, D-Arg⁸) Vasopressin, (Mpa¹, Ala⁴, D-Arg⁸) Vasopressin, (Mpa¹, Val⁴, D-Arg⁸) Vasopressin, (Mpa¹, Ile⁴, D-Arg⁸) Vasopressin, (Mpa¹, Leu⁴, D-Arg⁸) Vasopressin oder (Mpa¹, D-Gly⁸) Vasopressin umfasst.

10. Formulierung nach einem der Ansprüche 1 bis 9 zur Verwendung als Medikament.

11. Behälter, umfassend eine Formulierung nach einem der Ansprüche 1 bis 10 und eine Gebrauchsanleitung.

12. Formulierung nach Anspruch 8 zur Verwendung bei der therapeutischen und/oder prophylaktischen Behandlung von Hämorrhagie, wie etwa einer nachgeburtlichen Blutung.

13. Formulierung nach Anspruch 9 zur Verwendung bei der therapeutischen und/oder prophylaktischen Behandlung von Diabetes insipidus oder vasodilatorischem Schock.

14. Formulierung zur Verwendung nach Anspruch 12 oder 13, wobei das Medikament für eine Behandlung durch Verabreichung über den intravenösen, subkutanen oder intramuskulären Weg, den mukosalen Weg, oder einer Kombination davon ist.

15. Verwendung einer Kombination (i) eines Puffers und (ii) mindestens einer nicht toxischen Quelle von zweiwertigen Metallionen in einer Konzentration von mindestens 2 mM zur Stabilisierung einer wässrigen Formulierung von Oxytocin, Desamino-Oxytocin, Carbetocin, 4-Threonin-1-Hydroxy-Deaminooxytocin, 9-Deamidooxytocin, 7-D-Prolin-Oxytocin oder sein Deamino-Analogon, (2,4-Düsoleucin)-Oxytocin, 1-Desamino-1-Monocarba-E12-[Tyr(OMe)]-OT(dCOMOT), [Thr⁴-Gly⁷]-Oxytocin (TG-OT), Oxypressin, Deamino-6-Carba-Oxytoxin (dC60), (Mpa¹) Oxytocin, (Mpa¹, D-Tyr(Et)², Thr⁴, Orn⁸) Oxytocin, (Mpa¹, Ile²) Oxytocin, (Mpa¹, Ala²) Oxytocin, (Ile²) Oxytocin, Gly-(Leu⁴, Ile⁸) Oxytocin, (D-Asn⁵) Oxytocin, (D-Cys¹) Oxytocin, (Gly⁴) Oxytocin, (Leu⁴, D-Arg⁸) Oxytocin, (Mpa¹, Leu⁴, D-Arg⁸) Oxytocin, (Arg⁸) Oxytocin, (Ile⁸) Oxytocin, Ergometrin, Methylergometrin, Vasopressin, Desmopressin (1-Desamino-8-D-Arginin-Vasopressin oder DDAVP), Felypressin, Phenypressin, Lypressin (auch bekannt als Lysin-Vasopressin oder LVP), Ornipressin, Terlipressin, Pitressin (8-L-Arginin-Vasopressin, NC-1900, AVP₄₋₉), Desglycinamid-Arginin⁸-Vasopressin (DGAVP), d(CH₂)₅-Cys-d-Tyr(Et)-Arg-Val-Asn-Cys-Lys-Lys-Ethylendiamin (TA-LVP), d(CH₂)₅-Tyr(Me)-Arginin-Vasopressin, 4-Valin-8-D-Arginin-Vasopressin (VDAVP), [His^{1,6}]AVP, (Phe⁴, Arg⁸) Vasopressin, (Mpa¹, Arg⁸) Vasopressin, (Lys⁸) Vasopressin, Des-GlyNH₂-(Lys⁸) Vasopressin, Gly-Gly-Gly-(Lys⁸) Vasopressin, (Mpa¹, D-Arg⁸) Vasopressin, Des-GlyNH₂-(Mpa¹, D-Arg⁸) Vasopressin, (Mpa¹, Gly⁴, D-Arg⁸) Vasopressin, (Mpa¹, Ala⁴, D-Arg⁸) Vasopressin, (Mpa¹, Val⁴, D-Arg⁸) Vasopressin, (Mpa¹, Ile⁴, D-Arg⁸) Vasopressin, (Mpa¹, Leu⁴, D-Arg⁸) Vasopressin oder (Mpa¹, D-Gly⁸) Vasopressin.

## Revendications

1. Formulation aqueuse comprenant une quantité thérapeutiquement efficace d'oxytocine, désamino-oxytocine, carbétocine, 4-thréonine-1-hydroxy-désaminooxytocine, 9-désamidooxytocine, 7-D-proline-oxytocine ou son analogue désamino, (2,4-diisoleucine)-oxytocine, 1-désamino-1-monocarba-El2-[Tyr(OMe)]-OT(dCOMOT), [Thr⁴-Gly⁷]-oxytocine (TG-OT), oxypressine, désamino-6-carba-oxytocine (dC60), (MPa¹)-oxytocine, (MPa¹, D-Tyr(Et)², Thr⁴, Orn⁸)-oxytocine, (MPa¹, Ile²)-oxytocine, (MPa¹, Ala²)-oxytocine, (Ile²)-oxytocine, Gly-(Leu⁴, Ile⁸)-oxytocine, (D-Asn⁵)-oxytocine, (D-Cys¹)-oxytocine, (Gly⁴)-oxytocine, (Leu⁴, D-Arg⁸)-Oxytocine, (Mpa¹, Leu⁴, D-Arg⁸)-oxytocine, (Arg⁸)-oxytocine, (Ile⁸)-oxytocine, ergométrine, méthylergométrine, vasopressine, desmopressine (1-désamino-8-D-arginine-vasopressine ou DDAVP), félypressine, phénypressine, lypressine (également appelée lysine-vasopressine ou LVP), ornipressine, terlipressine, pitressine (8-L-arginine-vasopressine, NC-1900, AVP₄₋₉), desglycinamide-arginine⁸-vasopressine (DGAVP), d(CH₂)₅-Cys-d-Tyr(Et)-Arg-Val-Asn-Cys-Lys-Lys-éthylènediamine (TA-LVP), d(CH₂)₅-(Tyr(Me)-arginine-vasopressine, 4-valine-8-D-arginine-vasopressine (VDAVP), [His^{1,6}]AVP, (Phe⁴, Arg⁸)-vasopressine, (Mpa¹, Arg⁸)-vasopressine, (Lys⁸)-vasopressine, dés-GlyNH₂-(LyS⁸)-vasopressine, Gly-Gly-Gly-(Lys⁸)-vasopressine, (Mpa¹, D-Arg⁸)-vasopressine, dés-GlyNH₂-(Mpa¹, D-Arg⁸)-vasopressine, (Mpa¹, Gly⁴, D-Arg⁸)-vasopressine, (Mpa¹, Ala⁴, D-Arg⁸)-vasopressine, (Mpa¹, Val⁴, D-Arg⁸)-vasopressine, (Mpa¹, Ile⁴, D-Arg⁸)-vasopressine, (Mpa¹, Leu⁴, D-Arg⁸)-vasopressine ou (Mpa¹, D-Gly⁸)-vasopressine, un tampon et au moins une source non toxique d'ions métalliques divalents à une condition d'au moins 2 mM.

2. Formulation selon la revendication 1, comprenant des ions métalliques divalents à une concentration comprise entre 5 et 150 mM.

3. Formulation selon la revendication 1 ou 2 dans laquelle ladite source d'ions métalliques divalents est un sel de métal.

4. Formulation selon l'une quelconque des revendications 1 à 3, comprenant un tampon choisi dans le groupe constitué de tampon citrate, tampon acétate, tampon phosphate et tampon aspartate.

5. Formulation selon l'une quelconque des revendications 1 à 4, dans laquelle lesdits ions métalliques divalents sont choisis dans le groupe constitué de Ca²⁺, Mg²⁺, Cu²⁺ et Zn²⁺.

6. Formulation selon l'une quelconque des revendications 1 à 5, comprenant du tampon citrate et l'un ou plusieurs de Ca²⁺, Mg²⁺ et Zn²⁺.

7. Formulation selon l'une quelconque des revendications 1 à 5, comprenant un tampon aspartate et Zn²⁺.

8. Formulation selon l'une quelconque des revendications 1 à 7, **caractérisée en ce que** ladite formulation comprend de l'oxytocine, désamino-oxytocine, carbétocine, 4-thréonine-1-hydroxy-désaminooxytocine, 9-désamidooxytocine, 7-D-proline-oxytocine ou son analogue désamino, (2,4-diisoleucine)-oxytocine, 1-désamino-1-monocarba-E12-[Tyr(OMe)]-OT(dCOMOT), [Thr⁴-Gly⁷]-oxytocine (TG-OT), oxypressine, désamino-6-carba-oxytocine (dC60), (MPa¹)-oxytocine, (MPa¹, D-Tyr(Et)², Thr⁴, Orn⁸)-oxytocine, (MPa¹, Ile²)-Oxytocine, (MPa¹, Ala²)-oxytocine, (Ile²)-oxytocine, Gly-(Leu⁴, Ile⁸)-oxytocine, (D-Asn⁵)-oxytocine, (D-Cys¹)-oxytocine, (Gly⁴)-oxytocine, (Leu⁴, D-Arg⁸)-oxytocine, (Mpa¹, Leu⁴, D-Arg⁸)-oxytocine, (Arg⁸)-oxytocine ou (Ile⁸)-oxytocine, ergométrine, méthylergométrine.

9. Formulation selon l'une quelconque des revendications 1 à 7, **caractérisée en ce que** ladite formulation comprend de la vasopressine, desmopressine (1-désamino-8-D-arginine-vasopressine ou DDAVP), félypressine, phénypressine, lypressine (également appelée lysine-vasopressine ou LVP), ornipressine, terlipressine, pitressine (8-L-arginine-vasopressine, NC-1900, AVP₄₋₉), desglycinamide-arginine⁸-vasopressine (DGAVP), d(CH₂)₅-Cys-d-Tyr(Et)-Arg-Val-Asn-Cys-Lys-Lys-éthylènediamine (TA-LVP), d(CH₂)₅-(Tyr(Me)-arginine-vasopressine, 4-valine-8-D-arginine-vasopressine (VDAVP), [His^{1,6}]AVP, (Phe⁴, Arg⁸)-vasopressine, (Mpa¹, Arg⁸)-vasopressine, (Lys⁸)-vasopressine, dés-GlyNH₂(Lys⁸)-vasopressine, Gly-Gly-Gly-(Lys⁸)-vasopressine, (Mpa¹, D-Arg⁸)-vasopressine, dés-GlyNH₂-(Mpa¹, D-Arg⁸)-vasopressine, (Mpa¹, Gly⁴, D-Arg⁸)-vasopressine, (Mpa¹, Ala⁴, D-Arg⁸)-vasopressine, (Mpa¹, Val⁴, D-Arg⁸)-vasopressine, (Mpa¹, Ile⁴, D-Arg⁸)-vasopressine, (Mpa¹, Leu⁴, D-Arg⁸)-vasopressine ou (Mpa¹, D-Gly⁸)-vasopressine.

10. Formulation selon l'une quelconque des revendications 1 à 9 pour utilisation en tant que médicament.

11. Récipient comprenant une formulation selon l'une quelconque des revendications 1 à 10 et des instructions d'utilisation.

12. Formulation selon la revendication 8 pour utilisation dans le traitement thérapeutique et/ou prophylactique d'une hémorragie, telle qu'une hémorragie post-partum.

13. Formulation selon la revendication 9 pour utilisation dans le traitement thérapeutique et/ou prophylactique du diabète insipide ou d'un choc vasodilatateur.

14. Formulation pour utilisation selon la revendication 12 ou 13, **caractérisée en ce que** ledit médicament est pour un traitement par administration via la voie intraveineuse, sous-cutanée ou intramusculaire, la voie muqueuse, ou une combinaison de celles-ci.

15. Utilisation d'une combinaison de (i) un tampon, et (ii) au moins une source non toxique d'ions métalliques divalents à une concentration d'au moins 2 mM pour stabiliser une formulation aqueuse d'oxytocine, désamino-oxytocine, carbétocine, 4-thréonine-1-hydroxy-désaminooxytocine, 9-désamidooxytocine, 7-D-proline-oxytocine ou son analogue désamino, (2,4-diisoleucine)-oxytocine, 1-désamino-1-monocarba-E12-[Tyr(OMe)]-OT(dCOMOT), [Thr⁴-Gly⁷]-oxytocine (TG-OT), oxypressine, désamino-6-carba-oxytocine (dC60), (MPa¹)-oxytocine, (MPa¹, D-Tyr(Et)², Thr⁴, Orn⁸)-oxytocine, (MPa¹, Ile²)-oxytocine, (MPa¹, Ala²)-oxytocine, (Ile²)-oxytocine, Gly-(Leu⁴, Ile⁸)-oxytocine, (D-Asn⁵)-oxytocine, (D-Cys¹)-oxytocine, (Gly⁴)-oxytocine, (Leu⁴, D-Arg⁸)-oxytocine, (Mpa¹Leu⁴, D-Arg⁸)-oxytocine, (Arg⁸)-oxytocine, (Ile⁸)-oxytocine, ergométrine, méthylergométrine, vasopressine, desmopressine (1-désamino-8-D-arginine-vasopressine ou DDAVP), félypressine, phénypressine, lypressine (également appelée lysine-vasopressine ou LVP), ornipressine, terlipressine, pitressine (8-L-arginine-vasopressine, NC-1900, AVP₄₋₉), desglycinamide-arginine⁸-vasopressine (DGAVP), d(CH₂)₅-Cys-d-Tyr(Et)-Arg-Val-Asn-Cys-Lys-Lys-éthylènediamine (TA-LVP), d(CH₂)₅-(Tyr(Me)-arginine-vasopressine, 4-valine-8-D-arginine-vasopressine (VDAVP), [His^{1,6}]AVP, (Phe⁴, Arg⁸)-vasopressine, (Mpa¹, Arg⁸)-vasopressine, (Lys⁸)-vasopressine, dés-GlyNH₂(Lys⁸)-vasopressine, Gly-Gly-Gly-(Lys⁸)-vasopressine, (Mpa¹, D-Arg⁸)-vasopressine, dés-GlyNH₂-(Mpa¹, D-Arg⁸)-vasopressine, (Mpa¹, Gly⁴, D-Arg⁸)-vasopressine, (Mpa¹, Ala⁴, D-Arg⁸)-vasopressine, (Mpa¹, Val⁴, D-Arg⁸)-vasopressine, (Mpa¹, Ile⁴, D-Arg⁸)-vasopressine, (Mpa¹, Leu⁴, D-Arg⁸)-vasopressine ou (Mpa¹, D-Gly⁸)-vasopressine.
